# EUROPEAN PATENT SPECIFICATION

(11) **EP 1 061 901 B1**
(45) Date of publication and mention of the grant of the patent: **10.11.2004**
(21) Application number: 99939159.2
(22) Date of filing: 10.03.1999
(51) Int. Cl.: A61K 9/00, A61K 31/465

(54) **NICOTINE INHALER**
NIKOTININHALATOR
INHALEUR DE NICOTINE

(30) Priority: 11.03.1998 CA 2231968
(43) Date of publication of application: 27.12.2000
(73) Proprietor: NICO PUFF CORPORATION, Toronto, Ontario M5K 1H1 (CA)
(72) Inventor: PISKORZ, Hanna, Woodbrige, Ontario L4L 1L8 (CA)
(74) Representative: Shanks, Andrew
(86) International application number: PCT/CA1999/000188
(87) International publication number: WO 1999/045902

(56) References cited:
- EP-A- 0 148 748
- WO-A-97/12639
- WO-A-97/35562

## Description

### FIELD OF THE INVENTION

This invention relates to nicotine medicaments. In particular, the invention relates to a method of producing a nicotine medicament which is suitable for inhalation.

### BACKGROUND OF THE INVENTION

Smoking has been determined to be a contributory or causative factor in a number of diseases including respiratory diseases such as emphysema, chronic bronchitis, lung infections and lung cancer. Most regular smokers become addicted to, or dependent upon, the pharmacological effects of nicotine in tobacco smoke. Nicotine is rapidly absorbed across the blood/brain barrier and exerts a direct action on nicotine receptors in the spinal cord, autonomic ganglia and adrenal medulla.

Various nicotine replacement therapies have been developed. Some of these utilize a nicotine substitute. Nicotine substitutes generally contain nicotine in a solid form, in a vapour or in solution. For example, nicotine replacement therapy has included the use of nicotine gum. One disadvantage with nicotine gum is that lower steady state nicotine levels are achieved from chewing nicotine gum compared to smoking cigarettes and the rate of rise of blood nicotine levels is substantially lower as compared to smoking cigarettes. Further, the gum has been associated with gastrointestinal side effects, hiccups, mouth ulcers and sore throat. The amount of nicotine absorbed is also highly variable and is dependent upon the chewing and swallowing actions of the user over a prolonged period of time.

Nicotine patches have also been developed. One disadvantage of nicotine patches is that they have been associated with skin irritation at the site of application. Further, they result in a slow absorbtion of nicotine which may not be effective in satisfying a person's craving for cigarettes.

Self-propelled aerosols (also known as pressurized aerosols) which contain nicotine in solution have been proposed as cigarette substitutes. An example is the self-propelled formulation of Jacobs (United States Patent No. 4,635,651). As shown in Jacobs, these delivery systems contain a water based aerosol formulation and a propellent such as freon which are stored in a pressurized container. When actuated, Jacobs delivers nicotine and a solid carrier to the mouth of the user. Thus the aerosol created by Jacobs contains, in combination, a mixture of nicotine and the solid carrier. The nicotine is not formed as a composite part of the solid carrier. Further, the particle size of the aerosol created by Jacobs was variable. Therefore, the dose which is administered by using such pressurized aerosols may not be accurately controlled.

It has also been proposed to produce a dry powder inhaler for delivering a nicotine containing medicament via inhalation (see WO 97/12639). While nicotine formulations in the form of salts and complexes have been developed, there is still a need for a nicotine formulation which is adapted for inhalation into the alveoli and smaller airways of the lungs.

### SUMMARY OF THE INVENTION

Cigarette smoke is an aerosol comprising discrete particles of tar with which the nicotine is associated. The tar particles are of a size which makes them capable of travelling to the alveoli and lower airways of a person. Upon study, it has been determined that the nicotine is effectively conveyed to the alveoli and lower airways of a person by the tar particles. Current tobacco replacement therapies have not been effective in satisfying a person's craving for cigarettes. According to the instant invention, a nicotine formulation which more closely simulates cigarette smoke is provided which may be used with existing inhaler technology so as to improve the effectiveness of tobacco replacement or withdrawal therapies.

In accordance with the method of the instant invention, there is provided a composite material comprising discrete particles which are a mixture of nicotine and a carrier. As with cigarette smoke, the composite material is a physical combination of both the nicotine and the carrier. The carrier effectively provides a particle having a size and density such that it will be conveyed on inhalation to the alveoli and lower airways of a person. The nicotine is combined with the carrier such that it will be conveyed to the alveoli and lower airways of a person with the carrier. In contrast, in prior art formulations, the nicotine and the carrier were merely associated or aggregated with each other such that they separated from each other in the air stream. Thus the carrier did not act in general to transport a dose of the nicotine to the alveoli and lower airways of a person. In accordance with the instant invention, the nicotine and carrier form a composite material which are physically combined in such a way that they will not separate during inhalation.

In accordance with the method of the instant invention, there is provided a method of producing a nicotine medicament for use in an inhaler comprising:
(a) combining a nicotine formulation, a pharmaceutical grade sugar and a liquid carrier to produce a flowable mixture; and,
(b) drying the flowable mixture to produce a composite material at conditions to produce particles of the nicotine medicament suitable for delivery to the alveoli and lower airways of a person.

In one embodiment, the liquid carrier may comprise water. In another embodiment, the liquid carrier additionally comprises alcohol, particularly where the nicotine is a nicotine salt such as a nicotine sulphate or a nicotine tartrate. In this case, alcohol is added as a cosolvent, to expedite the solubility of the nicotine in the solution. In such a case, the liquid carrier preferably comprises a minor proportion of the alcohol and a major proportion of water. The ratio of alcohol to water in the liquid carrier may be from about 1:1 to 1:10, preferably from about 1:2 to 1:8 and more preferably from about 1:5 to 1:7 parts by weight.

The flowable mixture is preferably dried by spray drying. In one embodiment of the invention, the flowable mixture is atomized prior to being dried.

The flowable mixture is preferably dried at conditions to form substantially spherical particles. More preferably, the flowable mixture is dried at conditions to form spherical particles which have a dimpled surface. In one embodiment, the flowable mixture is dried at a temperature sufficiently high so that the liquid carrier is rapidly removed from the atomized particles of the flowable mixture in the spray drier.

An advantage of the instant invention is that the medicament particles produced by the method disclosed herein are well adapted for absorption into the bloodstream of a person via the alveoli and small airways of the lungs. The particles are a composite structure. Accordingly, the nicotine will not separate from the sugar (the carrier) during inhalation. Thus the sugar will convey the nicotine to the lungs in a manner to mimic cigarette smoke. By controlling the conditions at which the flowable mixture is spray dried, particles having a size from about 0.1 to about 5 µm, more preferably from about 0.5 to about 3 µm may be produced.

Nicotine, if it impacts upon the throat or upper airways of the person, may cause irritation. Thus, the method of the instant invention may be used to produce a powdered medicament formulation which, by inhalation, may reach the alveoli and smaller airways of a person's lungs without causing undue, and preferably, no irritation.

The method may also be used to produce particles which, not only are spherical, but have a uneven or a "dimpled" surface. The spherical shape of the dried particles reduces aggregation of the particles while in the inhaler, thus rendering it easier to aerosolize the particles upon inhalation by the user. Further, by having a dimpled surface, the aerodynamics of the medicament particles are improved whereby the particles may by more easily entrained in the air inhaled by the user.

### DESCRIPTION OF THE DRAWINGS

These and other advantages of the instant invention will be more fully and completely understood in accordance with the following description of a preferred embodiment of the invention, taken together with the drawings in which:
Figure 1 is a graph of nicotine concentration in a finished product made in accordance with the present invention versus nicotine concentration in the solution prior to being spray dried; and
Figure 2 is a graph of nicotine concentration in the finished product versus the ratio of nicotine to lactose in the solution prior to being spray dried.

### DESCRIPTION OF THE PREFERRED EMBODIMENT

According to the method of the instant invention, a composite material comprising nicotine and lactose is produced in a form suitable for inhalation by a user. In particular, the medicament comprises solid discrete flowable particles which may be entrained in the air inhaled by a person so as to travel to the alveoli and smaller airways of the lungs.

According to the method of the instant invention, a pharmaceutical grade sugar and nicotine are mixed with a liquid carrier so as to form a flowable mixture which may then be dried. The liquid carrier is an agent which mixes with the sugar and the nicotine to a degree sufficient to form a flowable mixture which may be rapidly dried such as in a spray drier. The nicotine, sugar and liquid carrier may be combined in any order.

The sugar is preferably selected from lactose, dextrose, glucose, maltose or combinations thereof, and is most preferably lactose. The sugar may be a natural or a synthetic sugar and may include analogs or derivatives of sugars. It will be appreciated that references herein are made to lactose, although one or more of the other sugars mentioned could similarly be employed. The lactose acts as a carrier and, therefore, any form of lactose approved as an excipient may be used. The lactose is preferably of a pharmaceutical grade such as CP, USP, NF, BP or BPC. The lactose which is used as a starting material is therefore in the form of a dry powder which is readily soluble in water.

The nicotine may be any form of nicotine which is soluble in or miscible with the liquid carrier. For example, the nicotine may be a nicotine base which, at room temperature, is a liquid that is miscible in water. Alternately, or in addition, the nicotine may be a salt which, at room temperature, is a solid. The nicotine base is typically an oil formulation. Preferably, the nicotine comprises nicotine base. The nicotine may be pharmacologically active analogs or derivatives of nicotine or substances which mimic the effect of nicotine, either alone or in combination with other active substances.

The liquid carrier may be any liquid or liquids with which the nicotine may be mixed and the lactose may be dissolved to form a flowable mixture which is preferably of a generally uniform composition. Nicotine bases are generally miscible in water and nicotine salt formulations are generally soluble in water. Further, lactose is soluble in water. Accordingly, whether the nicotine is a base and/or a salt formulation, the liquid carrier may comprise water. When a salt is used, the liquid carrier solubilizes the nicotine and the lactose. When a nicotine base is used, the liquid carrier solubilizes the lactose and mixes with the liquid base to create a generally uniform solution (eg. it is miscible with the liquid base). While water is the preferred liquid carrier, other liquids in combination with or in place of water may be used. For example, alternate liquids may be used, either by themselves or in combination to water, to solubilize the solid material or to disperse the nicotine base in the liquid carrier.

In a further preferred embodiment, the liquid carrier may comprise a mixture of alcohol and water. The water and the alcohol form an azeotropic mixture. Nicotine base formulations are readily soluble in an alcohol. However, the lactose is not soluble in the alcohol. Pursuant to this embodiment, the flowable mixture may comprise less water thus assisting in the rate of drying of the flowable mixture and/or the amount of water in the dried product.

Preferably, the alcohol is a primary alcohol. Further, the alcohol is preferably a lower alkyl alcohol (i.e. C₁ to C₅). A particularly preferred alcohol which may used as a solvent for the nicotine base solution is ethanol. The ethanol may be CP grade, and preferably, is, USP grade. However, it will be appreciated that it is preferable, where possible, to avoid the use of alcohol in the base solution.

This liquid carrier preferably contains an excess amount of water compared to alcohol where alcohol is necessary as a cosolvent. In such an embodiment, the mixture preferably comprises a minor proportion of alcohol and a major proportion of water. Where alcohol is required, the ratio of alcohol to water in the liquid carrier may be from about 1:1 to 1:10, preferably from about 1:2 to 1:8 and more preferably from about 1:5 to 1:7 parts by weight.

The liquid carrier (eg. water and/or alcohol) may be mixed with the nicotine to produce a liquid mixture to which the sugar may then be added. Accordingly, the lactose and a nicotine salt may be dissolved in water (and optionally a water/alcohol mixture) to form the flowable mixture. Alternately, the lactose may be dissolved in water (and optionally a water/alcohol mixture) and the nicotine base may be mixed with the water (and optionally a water/alcohol mixture) to form the flowable mixture. It will be appreciated that the nicotine, liquid carrier and sugar may be combined together in any desired order to produce the dry flowable mixture.

According to the preferred embodiment of this invention, the nicotine compound is added to the alcohol and mixed until a relatively consistent solution is achieved. Lactose is dissolved in water. Subsequently, the mixture of the nicotine in alcohol and added to the aqueous lactose solution and mixed until the flowable product is produced. The mixing may be conducted by any means known in the art.

The amount of liquid mixture which is utilized is sufficient to produce a flowable mixture. Pursuant to the preferred embodiment, the mixture is finely divided (such as passing the flowable mixture through an orifice) on entry to a spray dryer. Accordingly, the flowable mixture is preferably in the form of a liquid, such as a syrup or the like, which may readily be finely divided such as by passing the liquid through an atomizer (preferably a rotary atomizer).

The ratio of nicotine to lactose which is dissolved in the flowable mixture will vary upon the concentration of nicotine in the spray dried product. Due to product handling limitations, it is typical in the field that the carrier comprises a substantial portion of the weight of a powder medicament as compared to the active ingredient. The amount of lactose which is utilized, compared to the amount of nicotine, must be sufficient such that the spray dried product can be used in association with dry powder inhalers which are known in the art. Accordingly, the ratio of lactose to nicotine in the flowable mixture may vary from about 1:10 to about 10:1, more preferably from about 3:7 to about 3:2 and, most preferably, about 4:6 parts by weight. Further, the concentration of nicotine in the flowable mixture may vary from about 1 to about 10, more preferably from about 2 to about 5 and, most preferably, about 3 % (w/v, i.e. g/100 ml).

The flowable mixture is dried so as to produce particles which are sized so as to be able to travel to the alveoli and smaller airways of the lungs. Preferably, the particles have a particle size from about 0.1 to about 5 µm, more preferably from about 0.5 to about 5 µm and, most preferably from about 0.5 to about 5 µm based on the mass median aerodynamic diameter (MMAD) of the particles. The flowable mixture is preferably rapidly dried such as by using a spray drier. However, other drying techniques capable of producing appropriately sized particles (eg. the use of fluidized bed drying) may be used.

The flowable liquid is preferably rapidly dried so as to produce spherical or substantially spherical particles. Such particles may be achieved by using a rotary atomizer to feed the flowable liquid into a spray dryer.

The operating conditions of the spray dryer are adjusted so to produce particles which are sized so as to be able to travel to the alveoli and smaller airways of the lungs. The rotary atomizer may be operated at a liquid feed rate from about 2 to about 20, more preferably from 2 to about 10, and most preferably from about 2 to about 5 ml/min. The rotary atomizer may be operated from about 10,000 to about 30,000, more preferably from about 15,000 to about 25,000, and most preferably from about 20,000 to about 25,000 rpm.

The spray dryer is operated at temperatures sufficiently high to cause the liquid carrier to rapidly evolve without raising the temperature of the lactose and nicotine to a point at which these compounds commence to degrade. Accordingly, the spray dryer may be operated with an inlet temperature from about 120 to about 170°C and an outlet temperature from about 70 to about 100°C.

The medicament particles are spherical or of another aerodynamic shape. Such particles will tend not to aggregate when stored in a bulk form. Further, by evolving the liquid carrier sufficiently rapidly during the spray drying process, the medicament particles may be produced with an uneven or a "dimpled" surface. The uneven surface produces turbulence as the particles travel through the air, thus providing the particles with aerodynamic lift. This assists the particles to be entrained, and to remain entrained, in the air inhaled by a user thus improving the ability of the medicament particles to travel to the alveoli and smaller airways.

The following examples are intended to be illustrative only, and do not limit the scope of the invention.

### Examples

3g of nicotine and 27 g of lactose were added to 200 g of water. The mixture was stirred until the solution was clear (approximately 10 minutes). The mixture was spray dried in a Buchi Mini Spray Dryer 190, with an air flow rate of 500 ml/minute, an inlet temperature of 165°C and an outlet temperature of 87°C. The nicotine and lactose solution was fed into the atomizer at a rate of 7 ml/min. The results are set out in Table 1.

This experimental procedure was repeated under each of the sets of conditions set out in Table 1. Determination of the nicotine content in the nicotine lactose composite product was determined by using UV spectrophotometry at a wavelength of 262 nm. Particle size was determined using laser diffraction methods known in the art.

A concentration of 3% (w/v) of nicotine in solution, with a 4:6 ratio of nicotine to lactose (w/w) produced the highest concentration in the finished product. An air flow higher than 750 ml/min. resulted in a wet powder being produced which was detrimental to the flow characteristics.

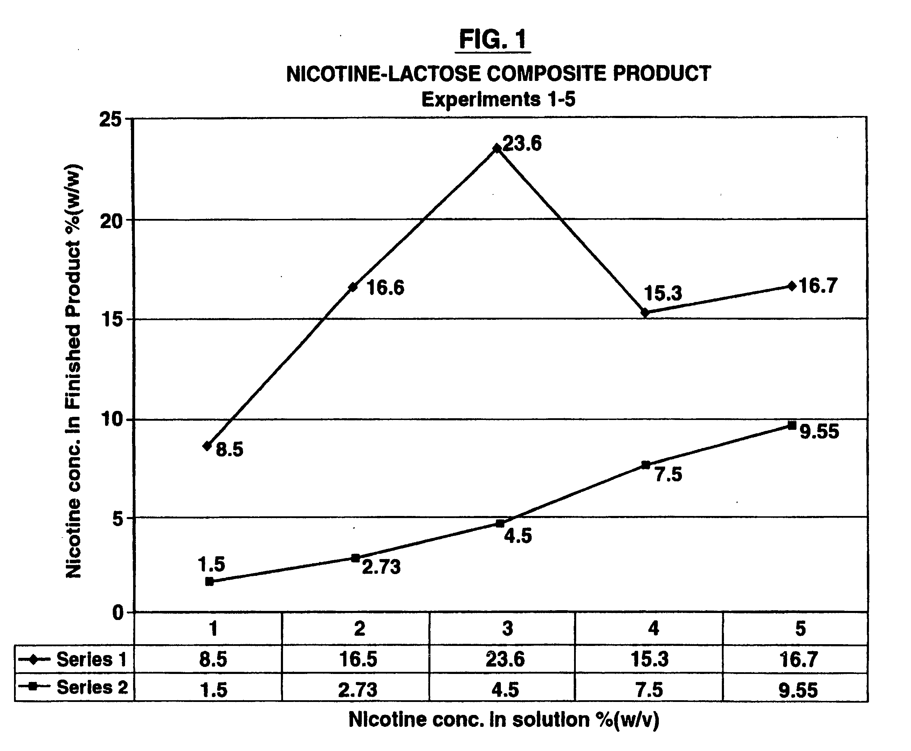

Figure 1 is a graph setting out the concentration of nicotine in the finished product as a function of the nicotine concentration in solution for experiments 1-5. "Series 1" is the concentration of nicotine in the finished product after spray drying. "Series 2" is the concentration of nicotine in solution prior to spray drying. It will be seen that a higher concentration of nicotine in solution did not always result in a higher concentration of nicotine in the finished product.

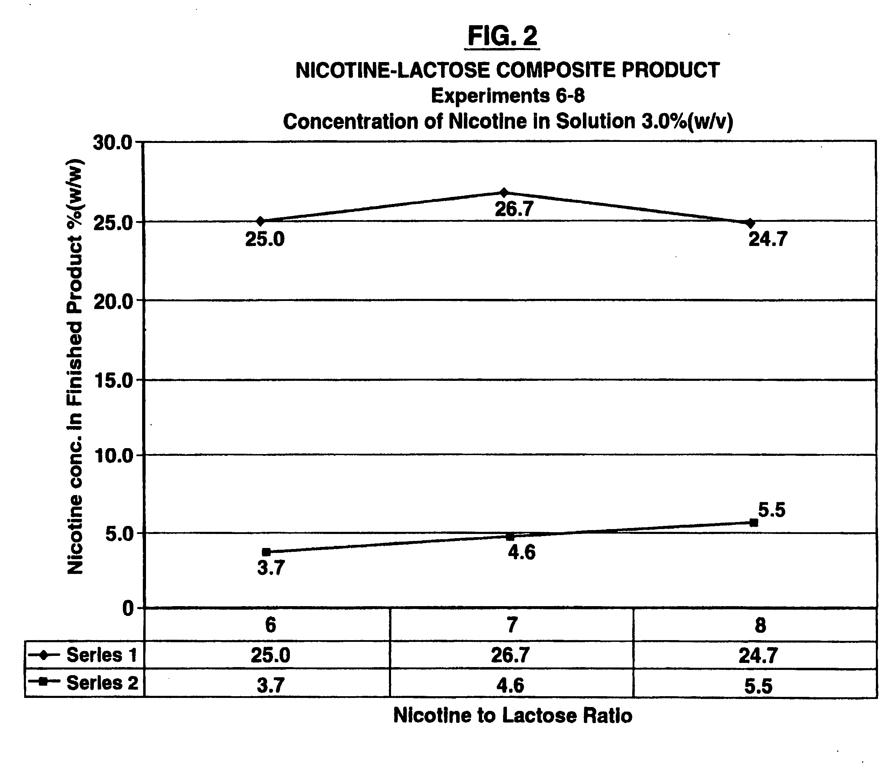

Figure 2 is a graph setting out the concentration of nicotine in the finished product as a function of the ratio of nicotine to lactose in the solution, for experiments 6-8. It will be seen that the higher nicotine to lactose ratio in solution did not necessarily produce a higher concentration of nicotine in the finished product. The highest ratio of nicotine to lactose in solution was determined to be approximately 3:7. "Series 1" in Figure 2 shows the concentration of nicotine in the finished product after spray drying, while "Series 2" shows the ratio of nicotine to lactose in solution prior to spray drying,

The results show that the highest concentration of nicotine in the finished product were achieved with a nicotine concentration of approximately 3% (w/v) in solution, and a nicotine:lactose ratio of approximately 4:6 in solution.

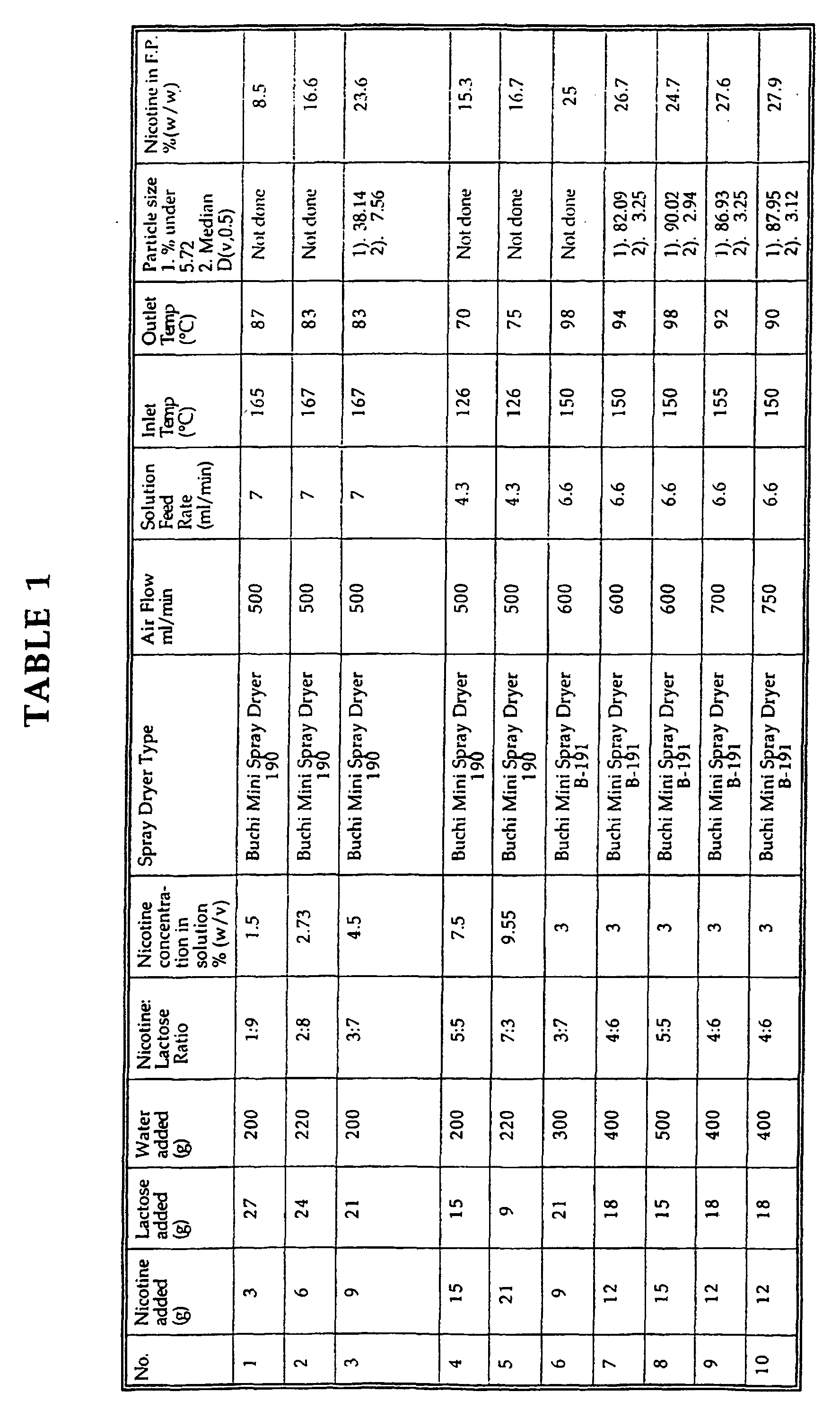

## Claims

1. A method of producing a nicotine medicament for use in an inhaler comprising:
(a) combining a nicotine formulation, a pharmaceutical grade sugar and a liquid carrier to produce a flowable mixture; and,
(b) drying the flowable mixture to produce a composite material at conditions to produce particles of the nicotine medicament suitable for delivery to the alveoli and lower airways of a person.

2. The method as claimed in claim 1 wherein the liquid carrier comprises water.

3. The method as claimed in claim 2 wherein the liquid carrier further comprises alcohol.

4. The method as claimed in claim 1 wherein the liquid carrier consists essentially of water.

5. The method as claimed in claim 2 wherein the nicotine composition comprises a nicotine base formulation.

6. The method as claimed in claim 2 wherein said nicotine composition comprises a nicotine salt formulation.

7. The method as claimed in claim 6 wherein the nicotine salt formulation comprises at least one salt selected from the group consisting of nicotine sulphates, nicotine tartrates and mixtures thereof.

8. The method as claimed in claim 3 wherein the alcohol is a C₁ to C₅ alkyl alchohol.

9. The method as claimed in claim 2 wherein the ratio of lactose to nicotine in the flowable mixture varies from about 1:10 to about 10:1 parts by weight

10. The method as claimed in claim 2 wherein the ratio of lactose to nicotine in the flowable mixture varies from about 3:7 to about 3:2 parts by weight.

11. The method as claimed in claim 9 wherein the concentration of nicotine in the flowable mixture varies from about 1 to about 10 w/v.

12. The method as claimed in claim 10 wherein the concentration of nicotine in the flowable mixture varies from about 2 to about 5 w/v.

13. The method as claimed in claim 1 wherein the flowable mixture is dried by spray drying.

14. The method as claimed in claim 13 wherein the flowable mixture is atomized prior to being spray dried.

15. The method as claimed in claim 1 wherein the flowable mixture is dried at conditions to form spherical particles.

16. The method as claimed in claim 1 wherein the flowable mixture is dried at conditions to form spherical particles which have a dimpled surface.

17. The method as claimed in claim 1 wherein the flowable mixture is dried at a temperature sufficiently high so that the liquid carrier is rapidly removed from the atomized particles of the flowable mixture.

18. The method as claimed in claim 1 wherein the particles are from about 0.1 to about 5 µm in diameter.

19. The method as claimed in any preceeding claim wherein step (a) of claim 1 comprises preparing a flowable solution consisting essentially of a nicotine formulation, a pharmaceutical grade sugar and a liquid carrier and the method further comprises packaging the composite material in a container for use with an inhaler suitable for delivering a medicament to the lungs.

20. A nicotine medicament for use in a dry powder inhaler comprising particles prepared from a solution of nicotine and a pharmaceutical grade sugar which are from about 0.1 to about 5 µm in diameter and which are physically joined together such that the nicotine and suger do not separate from each other upon inhalation.

21. The nicotine medicament as claimed in claim 20 wherein said nicotine comprises nicotine base.

22. The nicotine medicament as claimed in claim 20 wherein said particles consist essentialy of nicotine and sugar.

23. The nicotine medicament as claimed in claim 22 wherein said particles are spherical.

24. The nicotine medicament as claimed in claim 23 wherein said spherical particles have a dimpled surface.

25. The nicotine medicament as claimed in claim 20 wherein said nicotine medicament is prepared by rapidly drying a flowable solution of nicotine and sugar.

26. The nicotine medicament as claimed in claim 20 wherein the flowable solution is prepared by combining the nicotine and sugar with a liquid carrier comprising water.

27. The nicotine medicament as claimed in claim 26 wherein the nicotine medicament is prepared by spray drying.

28. The nicotine medicament as claimed in claim 25 wherein the ratio of lactose to nicotine in the flowable solution varies from about 1:10 to about 10:1 parts by weight and the concentration of nicotine in the flowable solution varies from about 1 to about 10 w/v.

29. The nicotine medicament as claimed in claim 25 wherein the ratio of lactose to nicotine in the flowable solution varies from about 3:7 to about 3:2 parts by weight and the concentration of nicotine in the flowable solution varies from about 2 to about 5 w/v.

## Patentansprüche

1. Verfahren zur Herstellung eines Nikotinmedikaments zur Verwendung in einem Inhalator, umfassend:
(a) Kombinieren einer Nikotinformulierung, eines Zuckers von pharmazeutischer Qualität und eines flüssigen Trägers, um ein fliessfähiges Gemisch herzustellen; und
(b) Trocknen des fliessfähigen Gemisches, um ein Kompositmaterial herzustellen, bei Bedingungen, so dass Partikel des Nikotinmedikaments, geeignet zum Transport zu den Alveolen und unteren Luftwegen einer Person, hergestellt werden.

2. Verfahren gemäss Anspruch 1, wobei der flüssige Träger Wasser umfasst.

3. Verfahren gemäss Anspruch 2, wobei der flüssige Träger des Weiteren Alkohol umfasst.

4. Verfahren gemäss Anspruch 1, wobei der flüssige Träger im Wesentlichen aus Wasser besteht.

5. Verfahren gemäss Anspruch 2, wobei die Nikotinzusammensetzung eine Nikotinbaseformulierung umfasst.

6. Verfahren gemäss Anspruch 2, wobei die Nikotinzusammensetzung eine Nikotinsalzformulierung umfasst.

7. Verfahren gemäss Anspruch 6, wobei die Nikotinsalzformulierung wenigstens ein Salz ausgewählt aus der Gruppe bestehend aus Nikotinsulfaten, Nikotintartraten und Gemischen davon umfasst.

8. Verfahren gemäss Anspruch 3, wobei der Alkohol ein C₁- bis C₅-Alkylalkohol ist.

9. Verfahren gemäss Anspruch 2, wobei in dem fliessfähigen Gemisch das Verhältnis von Laktose zu Nikotin von etwa 1:10-Gewichtsteilen bis etwa 10:1-Gewichtsteile variiert.

10. Verfahren gemäss Anspruch 2, wobei in dem fliessfähigen Gemisch das Verhältnis von Laktose zu Nikotin von etwa 3:7-Gewichtsteilen bis etwa 3:2-Gewichtsteile variiert.

11. Verfahren gemäss Anspruch 9, wobei in dem fliessfähigen Gemisch die Konzentration von Nikotin von etwa 1 bis etwa 10 w/v variiert.

12. Verfahren gemäss Anspruch 10, wobei in dem fliessfähigen Gemisch die Konzentration von Nikotin von etwa 2 bis etwa 5 w/v variiert.

13. Verfahren gemäss Anspruch 1, wobei das fliessfähige Gemisch durch Sprühtrocknen getrocknet wird.

14. Verfahren gemäss Anspruch 13, wobei das fliessfähige Gemisch zerstäubt wird, bevor es sprühgetrocknet wird.

15. Verfahren gemäss Anspruch 1, wobei das fliessfähige Gemisch bei Bedingungen getrocknet wird, so dass sphärische Partikel gebildet werden.

16. Verfahren gemäss Anspruch 1, wobei das fliessfähige Gemisch bei Bedingungen getrocknet wird, so dass sphärische Partikel gebildet werden, welche eine Oberfläche mit Grübchen aufweisen.

17. Verfahren gemäss Anspruch 1, wobei das fliessfähige Gemisch bei einer Temperatur getrocknet wird, welche ausreichend hoch ist, so dass der flüssige Träger rasch von den zerstäubten Partikeln des fliessfähigen Gemisches entfernt wird.

18. Verfahren gemäss Anspruch 1, wobei die Partikel einen Durchmesser von etwa 0,1 bis etwa 5 µm aufweisen.

19. Verfahren gemäss einem der vorhergehenden Ansprüche, wobei Schritt (a) gemäss Anspruch 1 umfasst:
das Herstellen einer im Wesentlichen aus einer Nikotinformulierung, einem Zucker von pharmazeutischer Qualität und einem flüssigen Träger bestehenden fliessfähigen Lösung,
und das Verfahren des Weiteren umfasst:
das Packen des Kompositmaterials in einen Behälter zur Verwendung mit einem Inhalator, geeignet zum Transportieren eines Medikaments zu den Lungen.

20. Nikotinmedikament zur Verwendung in einem Trockenpulverinhalator umfassend
von einer Lösung von Nikotin und einem Zucker von pharmazeutischer Qualität hergestellte Partikel,
welche einen Durchmesser von etwa 0,1 bis etwa 5 µm aufweisen, und
welche physikalisch derart in Kontakt gebracht worden sind, dass das Nikotin und der Zucker sich infolge Inhalation nicht voneinander trennen.

21. Nikotinmedikament gemäss Anspruch 20, wobei das Nikotin eine Nikotinbase umfasst.

22. Nikotinmedikament gemäss Anspruch 20, wobei die Partikel im Wesentlichen aus Nikotin und Zucker bestehen.

23. Nikotinmedikament gemäss Anspruch 22, wobei die Partikel sphärisch sind.

24. Nikotinmedikament gemäss Anspruch 23, wobei die sphärischen Partikel eine Oberfläche mit Grübchen aufweisen.

25. Nikotinmedikament gemäss Anspruch 20, wobei das Nikotinmedikament durch rasches Trocknen einer fliessfähigen Lösung von Nikotin und Zucker hergestellt worden ist.

26. Nikotinmedikament gemäss Anspruch 20, wobei die fliessfähige Lösung durch Kombinieren des Nikotins und des Zuckers mit einem Wasser umfassenden flüssigen Träger hergestellt worden ist.

27. Nikotinmedikament gemäss Anspruch 26, wobei das Nikotinmedikament durch Sprühtrocknen hergestellt worden ist.

28. Nikotinmedikament gemäss Anspruch 25, wobei in der fliessfähigen Lösung das Verhältnis von Laktose zu Nikotin von etwa 1:10-Gewichtsteilen bis etwa 10:1-Gewichtsteile variiert und die Konzentration von Nikotin in der fliessfähigen Lösung von etwa 1 bis etwa 10 w/v variiert.

29. Nikotinmedikament gemäss Anspruch 25, wobei in der fliessfähigen Lösung das Verhältnis von Laktose zu Nikotin von etwa 3:7-Gewichtsteilen bis etwa 3:2-Gewichtsteile variiert und die Konzentration von Nikotin in der fliessfähigen Lösung von etwa 2 bis etwa 5 w/v variiert.

## Revendications

1. Procédé de production d'un médicament à base de nicotine pour utilisation dans un inhaleur comprenant les étapes consistant à :
(a) combiner une formulation de nicotine, un sucre de qualité pharmaceutique et un support liquide pour produire un mélange fluide ; et
(b) sécher le mélange fluide pour produire une matière composée dans des conditions de production de particules de médicament à base de nicotine convenant pour apport dans les alvéoles et les voies respiratoires inférieures d'une personne.

2. Procédé selon la revendication 1, dans lequel le support liquide comprend de l'eau.

3. Procédé selon la revendication 2, dans lequel le support liquide comprend également de l'alcool.

4. Procédé selon la revendication 1, dans lequel le support liquide est composé essentiellement d'eau.

5. Procédé selon la revendication 2, dans lequel la composition de nicotine comprend une formulation à base de nicotine.

6. Procédé selon la revendication 2, dans lequel ladite composition de nicotine comprend une formulation de sels de nicotine.

7. Procédé selon la revendication 6, dans lequel la formulation de sels de nicotine comprend au moins un sel choisi dans le groupe constitué de sulfates de nicotine, tartrates de nicotine et leurs mélanges.

8. Procédé selon la revendication 3, dans lequel l'alcool est un alkyl alcool en C₁ à C₅.

9. Procédé selon la revendication 2, dans lequel le rapport du lactose à la nicotine dans le mélange fluide varie d'environ 1:10 à environ 10:1 parts en poids.

10. Procédé selon la revendication 2, dans lequel le rapport du lactose à la nicotine dans le mélange fluide varie d'environ 3:7 à environ 3:2 parts en poids.

11. Procédé selon la revendication 9, dans lequel la concentration de nicotine dans le mélange fluide varie d'environ 1 à environ 10 poids/volume.

12. Procédé selon la revendication 10, dans lequel la concentration de nicotine dans le mélange fluide varie d'environ 2 à environ 5 poids/volume.

13. Procédé selon la revendication 1, dans lequel le mélange fluide est séché par séchage par pulvérisation.

14. Procédé selon la revendication 13, dans lequel le mélange fluide est atomisé avant d'être séché par pulvérisation.

15. Procédé selon la revendication 1, dans lequel le mélange fluide est séché dans des conditions pour former des particules sphériques.

16. Procédé selon la revendication 1, dans lequel le mélange fluide est séché dans des conditions pour former des particules sphériques qui ont une surface ridée.

17. Procédé selon la revendication 1, dans lequel le mélange fluide est séché à une température suffisamment élevée, de sorte que le support liquide est rapidement éliminé des particules atomisées du mélange fluide.

18. Procédé selon la revendication 1, dans lequel les particules sont d'environ 0,1 à environ 5 µm en diamètre.

19. Procédé selon l'une quelconque des revendications précédentes, dans lequel l'étape (a) de la revendication 1 comprend la préparation d'une solution fluide composée essentiellement d'une formulation de nicotine, d'un sucre de qualité pharmaceutique et d'un support liquide et le procédé comprend également le conditionnement du produit composé dans un récipient pour utilisation avec un inhaleur adéquat pour apporter un médicament dans les poumons.

20. Médicament à base de nicotine pour utilisation dans un inhaleur de poudre sèche, comprenant des particules préparées à partir d'une solution de nicotine et d'un sucre de qualité pharmaceutique, qui sont d'environ 0,1 à environ 5 µm en diamètre et qui sont physiquement unies les unes aux autres, de manière que la nicotine et le sucre ne se séparent pas l'un de l'autre lors de l'inhalation.

21. Médicament à base de nicotine selon la revendication 20, dans lequel ladite nicotine comprend une base de nicotine.

22. Médicament à base de nicotine selon la revendication 20, dans lequel lesdites particules sont composées essentiellement de nicotine et de sucre.

23. Médicament à base de nicotine selon la revendication 22, dans lequel lesdites particules sont sphériques.

24. Médicament à base de nicotine selon la revendication 23, dans lequel lesdites particules sphériques ont une surface ridée.

25. Médicament à base de nicotine selon la revendication 20, dans lequel ledit médicament à base de nicotine est préparé en séchant rapidement une solution fluide de nicotine et de sucre.

26. Médicament à base de nicotine selon la revendication 20, dans lequel ladite solution fluide est préparée en combinant la nicotine et le sucre avec un support liquide comprenant de l'eau.

27. Médicament à base de nicotine selon la revendication 26, dans lequel le médicament à base de nicotine est préparé par séchage par pulvérisation.

28. Médicament à base de nicotine selon la revendication 25, dans lequel le rapport du lactose à la nicotine dans la solution fluide varie d'environ 1:10 à environ 10:1 parts en poids et la concentration de nicotine dans la solution fluide varie d'environ 1 à environ 10 poids/volume.

29. Médicament à base de nicotine selon la revendication 25, dans lequel le rapport du lactose à la nicotine dans la solution fluide varie d'environ 3:7 à environ 3:2 et la concentration de nicotine dans la solution fluide varie d'environ 2 à environ 5 poids/volume.
